# EUROPEAN PATENT SPECIFICATION

(11) **EP 2 527 823 B1**
(45) Date of publication and mention of the grant of the patent: **18.04.2018**
(21) Application number: 11734675.9
(22) Date of filing: 19.01.2011
(51) Int. Cl.: G01N 25/56, A61F 5/44, A61F 13/42, A61F 13/49, G01N 27/02

(54) **EXCRETION DETECTION DEVICE AND ABSORBENT ARTICLE**
AUSSCHEIDUNGSDETEKTOR UND SAUGFÄHIGER ARTIKEL
DÉTECTEUR D'EXCRÉTION ET ARTICLE ABSORBANT

(30) Priority: 19.01.2010 JP 2010009301
(43) Date of publication of application: 28.11.2012
(73) Proprietor: Unicharm Corporation, Ehime 799-0111 (JP)
(72) Inventor: SUZUKI, Miou, Kanonji-shi Kagawa 769-1602 (JP); FUJIOKA, Yoshihisa, Kanonji-shi Kagawa 769-1602 (JP)
(74) Representative: Dolleymores
(86) International application number: PCT/JP2011/050867
(87) International publication number: WO 2011/090069

(56) References cited:
- JP-A- 2000 185 067
- JP-A- 2000 329 732
- JP-A- 2002 022 687
- JP-A- 2002 143 199
- JP-A- 2002 301 098
- JP-A- 2005 000 602
- JP-A- 2009 210 533
- US-A1- 2009 315 728

## Description

### [Technical Field]

The present invention relates to an excretion detection device, by which the excretion of bodily waste from a living body is detected, and an absorbent article including such an excretion detection device.

### [Background Art]

As regards the absorbent article such as a disposable diaper, an excretion detection device configured to detect the excretion of urine or stool from a wearer (living body) of the absorbent article is known. The excretion detection device is loaded in the absorbent article, and upon detection of the excretion of urine or stool from the wearer, the excretion detection device reports the excretion to the outside.

In such an excretion detection device, a method of electrification, by bringing the bodily waste of the wearer in contact with electrodes configured from materials having different ionization tendencies, is known. That is, there has been proposed a device configured to actuate an alarm such as a buzzer by making use of the principle of a voltaic cell rather than using a battery (see Patent Document 1 and Patent Document 2).

In the excretion detection device disclosed in Patent Document 1, copper or zinc is used as materials of the electrodes. Furthermore, in the excretion detection device disclosed in Patent Document 2, there are described that electrification through the use of the bodily waste (urine) and a battery are put to use together, and gold, silver, copper, or aluminum can be used as materials of the electrodes.

### [Prior Art Document]

### [Patent Document]

[PATENT DOCUMENT 1]: Japanese Patent Application Publication No. 2002-277435 (Page 3, Fig. 4)
[PATENT DOCUMENT 2]: Japanese Patent Application Publication No. 2006-296566 (Page 4, Fig. 1)
JP 2000 329732 A relates to an excretion detection device having a cathode, a dry electrolyte, an anode and a transmitter. When excrement is excreted, moisture is applied to the dried electrolyte such that a chemical battery is provided which can generate electric power, and radio waves are radiated from the transmitter.
US 2009/315728 A1 discloses a method of reducing sensor corrosion in absorbent articles. The method includes providing a monitor adapted to electrically connect with a wetness sensor integrated with an absorbent article. The wetness circuit generally includes one or more conductors, a voltage source and a measuring device.

### [Summary of the Invention]

However, the aforementioned conventional excretion detection device has the following problem. That is, although the electrodes are in contact with the bodily waste of the wearer, if the pH of the bodily waste is high, corrosion of the electrodes speeds up. Particularly, the pH of urine of elderly persons is often high due to urinary tract infections and the like, and therefore, when the electrodes come in contact with such urine having a high pH, the electrodes corrode fast, and a sufficient electrification time cannot be secured, which is a point of concern.

Though the use of electrodes with high corrosion resistance is thought of, from the viewpoint of the use of such electrodes in sanitary products as well as the manufacturing cost, this is not a practical solution.

Therefore, an object of the present invention is to provide an excretion detection device by which a sufficient electrification time can be secured even in the case of contact with urine with a high pH, without significantly increasing the manufacturing cost, and also to provide an absorbent article having such an excretion detection device.

In order to solve the above-mentioned problem, the present invention provides the absorbent article of independent claim 1. The dependent claim specifies a preferred but optional feature.

The present invention includes an excretion detection device (excretion detection device 100) configured to detect excretion of bodily waste (bodily waste Ex, e.g., urine and stool) from a living body (wearer W), comprising: a power supply unit (power supply unit 160) having electrodes (electrode 121, electrode 122) configured by using materials with different ionization tendencies; a neutralizer retention unit (neutralizer retention unit 112) configured to retain a neutralizer (neutralizer N) for reducing the hydrogen ion index of the bodily waste; and a notification unit (radio transmission unit 180) operated by the electric power generated by the power supply unit, and configured to notify the excretion from the living body to outside the excretion detection device, wherein the electrodes are provided at a position where the electrodes can be in contact with the bodily waste, and the neutralizer retention unit is provided at a position where the neutralizer can be in contact with the bodily waste.

The excretion detection device comprises a solution retention unit (electrolyte solution retention unit 111) configured to retain an electrolyte solution (electrolyte solution WT), wherein the electrodes are provided at a position where the electrodes are contactable with the bodily waste and the electrolyte solution, the electrodes being adapted to be in contact with the electrolyte solution before the excretion of the bodily waste. Also, the power supply unit is is configured to obtain electric power when the bodily waste or the electrolyte solution is arranged between the pair of electrodes.

According to the present invention, the neutralizer retention unit is provided so as to be positioned at the upper side of the electrodes during the use of the excretion detection device.

According to the present invention, the neutralizer retention unit is preferably adjacent to the solution retention unit.

According to the present invention, the neutralizer is preferably adapted to seep out from the neutralizer retention unit by performing a predetermined operation for the neutralizer retention unit, the neutralizer being contactable with the bodily waste.

According to the present invention, the electrolyte solution is preferably adapted to seep out from the solution retention unit by performing a predetermined operation (e.g., pressing) for the solution retention unit, the electrolyte solution being contactable with the electrodes.

An absorbent article (e.g., disposable diaper 10A), worn by a living body and configured to absorb the bodily waste from the living body, includes an excretion detection device as described above.

According to the present invention, the absorbent article comprises: a topsheet (topsheet 11) that is in contact with the living body and allows the passage of liquids; and an absorber (absorber 13) configured to absorb the bodily waste, and the excretion detection device is provided between the topsheet and the absorber.

According to the present invention, it is possible to provide an excretion detection device by which a sufficient electrification time can be secured even in the case of contact with urine with a high pH, without increasing the manufacturing cost substantially, and also to provide an absorbent article having such an excretion detection device.

### [Brief Description of the Drawings]

[Fig. 1] Fig. 1 is a diagram showing an entire schematic configuration of an excretion management system 1 according to an embodiment of the present invention.
[Fig. 2] Fig. 2 is a developed plan view of a disposable diaper 10A in which an excretion detection device 100 according to the embodiment of the present invention is embedded, and is also a schematic illustration of the appearance of the excretion detection device 100.
[Fig. 3] Fig. 3 is a diagram showing a functional block configuration of the excretion detection device 100 according to the embodiment of the present invention.
[Fig. 4] Fig. 4 is a diagram showing the excretion management flow using the excretion management system 1 according to the embodiment of the present invention.
[Fig. 5] Fig. 5 is a schematic cross-sectional view of the disposable diaper 10A along an F5-F5 line of Fig. 2.

### [Description of Embodiments]

Next, an embodiment of an excretion detection device and an absorbent article according to the present invention will be described with reference to drawings. In the following description of the drawings, the same or similar reference numerals are used to designate the same or similar parts. It will be appreciated that the drawings are schematically shown and the ratio and the like of each dimension are different from the real ones.

Accordingly, specific dimensions should be determined in consideration of the explanation below. Moreover, among the drawings, the respective dimensional relations or ratios may differ.

### (1) Entire schematic configuration of excretion management system

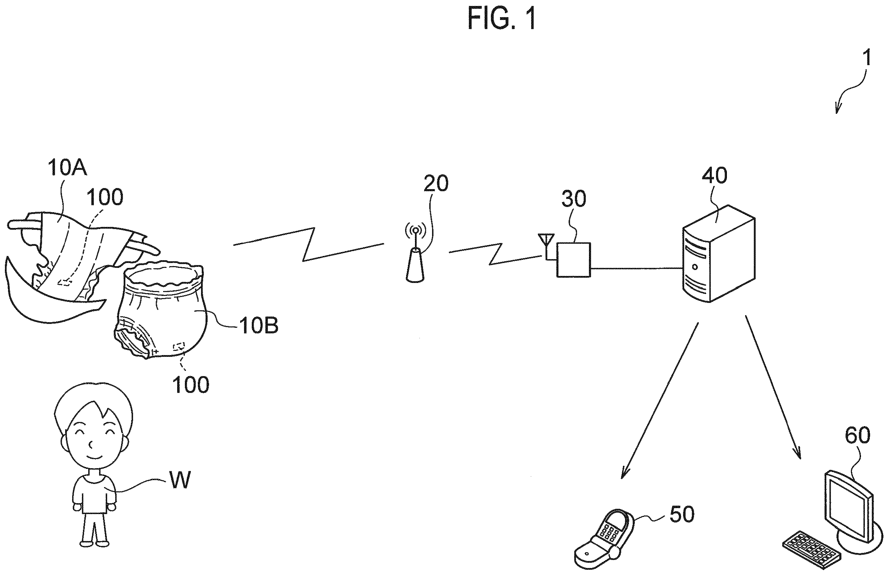

Fig. 1 is a diagram showing an entire schematic configuration of an excretion management system 1 according to the present embodiment.
As shown in Fig. 1, the excretion management system 1 is configured by a radio relay station 20, an excretion management server 40, and an excretion detection device 100.

A disposable diaper 10A and a disposable diaper 10B are absorbent articles worn by a wearer W (living body), and to absorb a bodily waste such as urine or stool from the wearer W. The disposable diaper 10A and the disposable diaper 10B are equipped with the excretion detection device 100 configured to detect excretion of bodily waste. Specifically, the excretion detection device 100 is loaded in an open-type (tape-type) disposable diaper 10A and a pant-type disposable diaper 10B.

The excretion detection device 100 detects the excretion of bodily waste from the wearer W. The excretion detection device 100 is configured by an active-type IC tag that can transmit a radio signal to the radio relay station 20, a temperature sensor, and the like, and repetitively transmits a radio signal including the temperature information (environment information) within the disposable diaper, at a predetermined period. The detailed configuration of the excretion detection device 100 is described later.

The radio relay station 20 converts the radio signal received from the excretion detection device 100 to a radio signal in accordance with a predetermined communication system. In the present embodiment, the radio relay station 20 converts the radio signal received from the excretion detection device 100 to a radio signal of the Personal Handy-phone (PHS system), and then transmits the radio signal to a radio base station 30. The radio relay station 20 is a rectangular parallelepiped with one side of a few centimeters or less, which can be installed in the bed as a name tag, and can be installed on to the wearer W. In order to maintain radio communication between the radio relay station 20 and the excretion detection device 100, the radio relay station 20 and the excretion detection device 100 must be positioned within a fixed distance.

The radio base station 30 is configured to receive a radio signal from the radio relay station 20. In the present embodiment, the radio base station 30 transmits and receives a radio signal in accordance with the PHS system. The radio base station 30 is connected to the excretion management server 40 via a wire communication network (not shown in the figure).

The excretion management server 40 stores a plurality of change patterns of the environmental information (such as the temperature, humidity, and odor) concerning urination and defecation. The excretion management server 40 determines the existence or non-existence of urination or defecation by the wearer W based on the temperature information transmitted repetitively from the excretion detection device 100 via the radio relay station 20 and the radio base station 30. Upon determining the occurrence of urination or defecation, the excretion management server 40 transmits an email indicating the excretion to a pre-registered cellular phone terminal 50 and a personal computer 60. When the excretion management server 40 transmits such an email to a pre-registered address in the cellular phone terminal 50 and the personal computer 60, a guardian (caretaker) of the wearer W, who checks the email can quickly take an appropriate measure, such as changing the disposable diaper.

### (2) Layout position and structure of excretion detection device

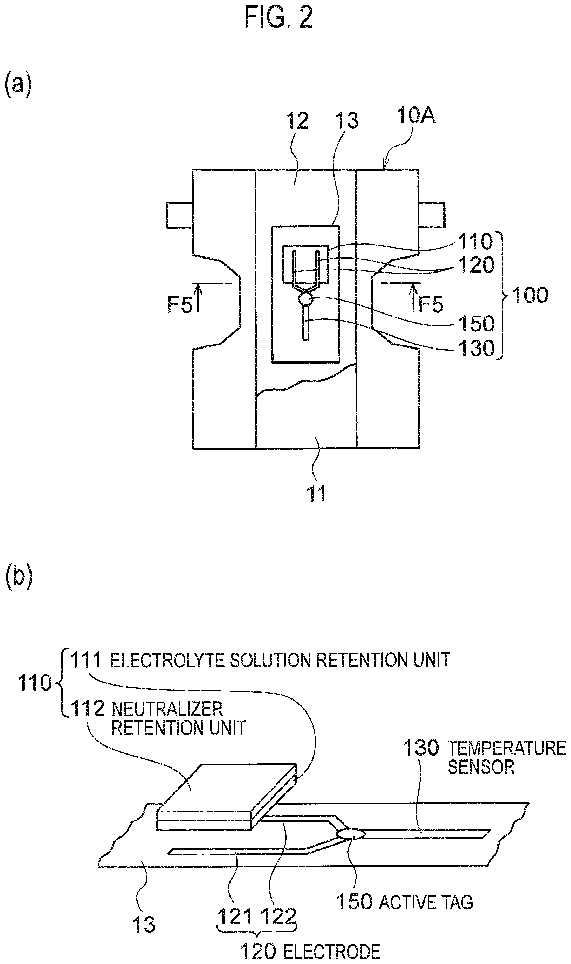

Fig. 2 (a) is a developed plan view of the disposable diaper 10A in which the excretion detection device 100 is embedded. Fig. 2 (b) is a schematic illustration of the appearance of the excretion detection device 100.

As shown in Fig. 2 (a), the disposable diaper 10A includes a topsheet 11, a backsheet 12, and an absorber 13. The topsheet 11 is in contact with the skin of the wearer W (living body). The topsheet 11 is configured from a material that allows liquids such as urine to pass through. The backsheet 12 is in contact with the clothing of the wearer W. The backsheet 12 is configured from a material that does not allow liquids to pass through. The absorber 13 absorbs bodily waste, such as urine or stool. The absorber 13 is configured from hydrophilic fibers such as ground pulp. The disposable diaper 10A can be manufactured according to a well-known method (for example, Japanese Unexamined Patent Publication No. JP2003-339771).

The excretion detection device 100 is provided between the topsheet 11 and the absorber 13 in order to be sure that the stool, which has a higher viscosity as compared to urine, is brought in contact with an electrode unit 120 of the excretion detection device 100. Furthermore, even in the case of the open-type disposable diaper 10B, the excretion detection device 100 is desired to be provided between the topsheet 11 and the absorber 13. The disposable diaper 10B can be also manufactured according to a well-known method (for example, Japanese Patent Application Publication No. JP2005-58755).

The excretion detection device 100 includes a solution and neutralizer retention unit 110, the electrode unit 120, a temperature sensor 130, and an active tag 150. The solution and neutralizer retention unit 110 has an electrolyte solution retention unit 111 and a neutralizer retention unit 112. The electrolyte solution retention unit 111 retains an electrolyte solution. The neutralizer retention unit 112 retains a neutralizer for reducing the hydrogen ion index (pH) of the bodily waste. In the present embodiment, the neutralizer retention unit 112 is provided adjacent to the electrolyte solution retention unit 111. Specifically, the neutralizer retention unit 112 is provided adjacent to the upper side of the electrolyte solution retention unit 111.

The electrode unit 120 is provided at the lower side of the electrolyte solution retention unit 111 (towards the absorber 13). The temperature sensor 130 is provided on the opposite side of an electrode 121 and an electrode 122, with the active tag 150 being disposed, as the reference, therebetween, in order to prevent coming in contact with the electrolyte solution seeping out from the electrolyte solution retention unit 111 and the neutralizer seeping out from the neutralizer retention unit 112. The electrode 121 and the electrode 122 are provided at a position where the electrodes can come in contact with the bodily waste from the wearer W and the electrolyte solution seeping out from the electrolyte solution retention unit 111. That is, the electrode 121 and the electrode 122 can be brought in contact with the electrolyte solution seeping out from the electrolyte solution retention unit 111 before the excretion of urine and the like.

The active tag 150 is connected to the electrode unit 120 and the temperature sensor 130, and is provided at a position where the active tag 150 does not overlap the solution and neutralizer retention unit 110.

### (3) Functional block configuration of the excretion detection device 100

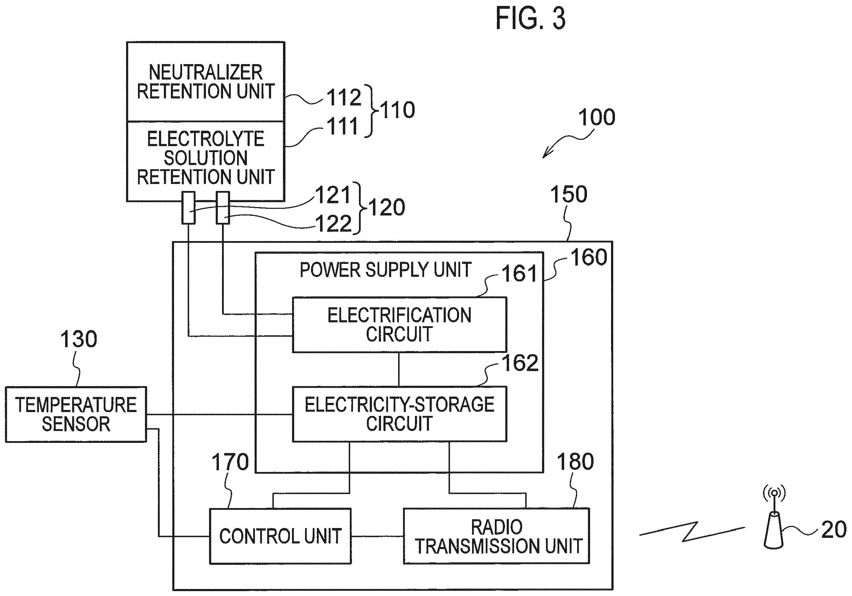

Fig. 3 is a diagram showing the functional block configuration of the excretion detection device 100. As shown in Fig. 3, the excretion detection device 100 includes the solution and neutralizer retention unit 110, the electrode unit 120, the temperature sensor 130, and the active tag 150. Furthermore, the active tag 150 is configured by a power supply unit 160, a control unit 170, and a radio transmission unit 180.

As described above, the solution and neutralizer retention unit 110 has the electrolyte solution retention unit 111 and the neutralizer retention unit 112.

The electrolyte solution retention unit 111 retains an electrolyte solution prepared by dissolving an electrolyte in a solvent such as water. For example, the electrolyte solution retention unit 111 can use an aqueous solution including approx. 1 wt.% to 5 wt.% of sodium chloride (NaCl). That is, an NaCl aqueous solution similar to the urinary constituent is used as the electrolyte solution. The electrolyte solution retention unit 111 is a bag-shaped container that can store the liquid, and in the present embodiment, the electrolyte solution retention unit 111 is configured from a thermoplastic film of polyolefin.

By performing a predetermined operation for the electrolyte solution retention unit 111, for example, by pressing the electrolyte solution retention unit 111 from the topsheet 11 side, the electrolyte solution is made to seep out from the electrolyte solution retention unit 111. That is, by pressing the electrolyte solution retention unit 111, the bag-shaped electrolyte solution retention unit 111 configured from a thermoplastic film is torn, and the electrolyte solution seeps out near the periphery of the electrode unit 120. As a result, the electrolyte solution comes in contact with the electrode 121 and the electrode 122.

The electrolyte solution retention unit 111 stores only solvents such as water, and powdered sodium chloride may be placed in the proximity of the electrolyte solution retention unit 111. In such a case, the powdered sodium chloride dissolves in the water seeping out from the electrolyte solution retention unit 111 to result in an electrolyte solution. This case also applies to a case where an electrolyte solution is retained by the electrolyte solution retention unit 111.

The neutralizer retention unit 112 retains a neutralizer, such as citric acid, for reducing the pH of the bodily waste. Besides citric acid, tartaric acid and succinic acid may also be used as the neutralizer. The neutralizer retention unit 112 is provided at a position where the neutralizer can be in contact with the bodily waste. Specifically, the neutralizer retention unit 112 is provided to be positioned at the upper side of the electrode unit 120 (towards the topsheet 11) during the use of the excretion detection device 100.

Same as the electrolyte solution retention unit 111, the neutralizer retention unit 112 is configured from a thermoplastic film of polyolefin. Furthermore, by performing a predetermined operation for the neutralizer retention unit 112, for example, by pressing the electrolyte solution retention unit 111 from the topsheet 11 side, the neutralizer is made to seep out from the neutralizer retention unit 112. That is, by pressing the neutralizer retention unit 112, the bag-shaped neutralizer retention unit 112 configured from a thermoplastic film is torn, and the neutralizer seeps out near the periphery of the bodily waste. As a result, the neutralizer comes in contact with the bodily waste. Therefore, the corrosion of the electrode 121 and the electrode 122 is prevented even when the bodily waste is urine with a high pH, for example.

The electrode unit 120 is provided at a position where the electrode unit 120 can be in contact with the bodily waste. Specifically, the electrode unit 120 is provided at the lower side of the solution and neutralizer retention unit 110 (towards the absorber 13), and on the absorber 13. The pair of electrode 121 and electrode 122 configuring the electrode unit 120 are connected to the power supply unit 160. The electrode 121 and the electrode 122 are configured using materials having different ionization tendencies. In the present embodiment, a combination of aluminum and silver (each being a mixture combined with carbon) is used as the electrode 121 and the electrode 122. That is, the power supply unit 160 having such an electrode unit 120 functions as a so-called voltaic cell through the interposition of the electrolyte solution or the bodily waste between the electrode 121 and the electrode 122. A voltaic cell is a cell that generates electric current by moving electrons through the immersion of electrodes using a metal with a large ionization tendency and a metal with a small ionization tendency, in an electrolyte solution.

As described above, the electrode 121 and the electrode 122 can be brought in contact with the electrolyte solution seeping out from the electrolyte solution retention unit 111 before the excretion of urine and the like. Therefore, a difference in potential occurs between the electrode 121 and the electrode 122 due to the electrolyte solution prior to excretion, and due to the electrolyte solution or the bodily waste after excretion, and electromotive force is obtained.

The temperature sensor 130 is configured to detect the environmental information that changes as a result of excretion from the wearer W. Specifically, in the disposable diaper 10A, the temperature sensor 130 detects the temperature of the region where the bodily waste is excreted (the region in which the absorber 13 is provided) as the environment information. The temperature sensor 130 is operated by the electric power generated by the power supply unit 160.

In the present embodiment, a thin-film thermistor (hereinafter, thermistor) is used as the temperature sensor 130. In the temperature sensor 130, a synthetic-resin film on which the electrode for detecting urine or stool, and the electrode for supplying electric power to the thermistor are marked by a conductive ink is used. A thermistor that is easily affected by the surrounding temperature at which the heat capacity is less, for example, the ET-103 thermistor manufactured by SEMITEC CORPORATION, is desired to be used as the temperature sensor 130.

The active tag 150 is an active-type IC tag with which the electrode unit 120 and the temperature sensor 130 are connected. That is, the active tag 150 can transmit a radio signal without depending on another radio communication device, for example.

The power supply unit 160 has an electrification circuit 161 and an electricity-storage circuit 162. The electrode 121 and the electrode 122 are connected to the electrification circuit 161. The electrification circuit 161 is electrified due to the difference in potential that occurs between the electrode 121 and the electrode 122, and stores the generated electric power in the electricity-storage circuit 162. The electricity-storage circuit 162 is configured from a capacitor, for example.

The control unit 170 is operated by the electric power generated by the power supply unit 160. Specifically, based on the value output from the temperature sensor 130, the control unit 170 generates data indicating the temperature information, and then outputs the generated data to the radio transmission unit 180.

The radio transmission unit 180 is operated by the electric power generated by the power supply unit 160. The radio transmission unit 180 notifies the temperature information detected by the temperature sensor 130 to outside the excretion detection device 100. In the present embodiment, the radio transmission unit 180 configures a notification unit configured to notify excretion from the wearer to outside the excretion detection device 100.

Specifically, the radio transmission unit 180 transmits a radio signal including the data indicating the temperature information towards the radio relay station 20. In the present embodiment, the radio transmission unit 180 repetitively transmits radio signals at a predetermined period (for example, 10 seconds).

As described above, because the excretion detection device 100 functions as a voltaic cell before and after excretion, the excretion detection device 100 is a battery-less type device in which a battery is not loaded for operating the temperature sensor 130, the control unit 170, and the radio transmission unit 180.

### (4) Excretion management method using the excretion management system

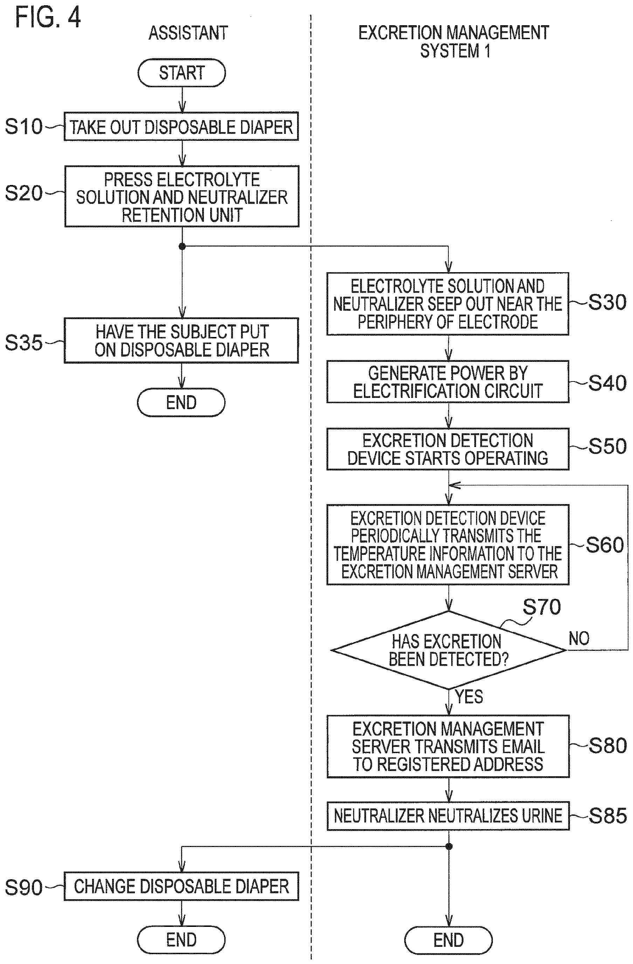

Fig. 4 shows the excretion management flow using the aforementioned excretion management system 1. Specifically, Fig. 4 shows the flow from the detection of urination or defecation, by the excretion detection device 100, from the wearer W wearing the disposable diaper 10A (or the disposable diaper 10B), up to the transmission of an email notifying the cellular phone terminal 50 and the personal computer 60 of this effect.

As shown in Fig. 4, in step S10, a guardian and a caretaker (hereinafter, assistant) assisting subjects such as care-receivers in wearing the disposable diaper 10A to take out the disposable diaper 10A from the package, for example.

In step S20, the assistant presses the solution and neutralizer retention unit 110 embedded at the lower side of the topsheet 11 of the disposable diaper 10A. As a result of such an operation by the assistant, the bag-shaped electrolyte solution retention unit 111 and the neutralizer retention unit 112 are torn.

In step S30, the electrolyte solution (sodium chloride solution) seeps out from the electrolyte solution retention unit 111 near the periphery of the electrode unit 120. Furthermore, the neutralizer seeps out from the neutralizer retention unit 112 near the periphery of the electrode unit 120.

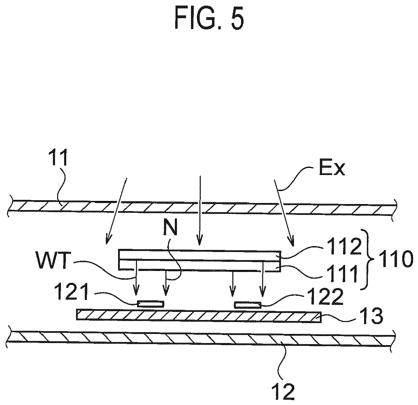

Here, the state in which the electrolyte solution and the neutralizer seep out near the periphery of the electrode unit 120 is explained with reference to Fig. 5. Fig. 5 is a schematic cross-sectional view of the disposable diaper 10A along an F5-F5 line of Fig. 2. As shown in Fig. 5, when a portion of the solution and neutralizer retention unit 110 is pressed, the bag-shaped electrolyte solution retention unit 111 and the neutralizer retention unit 112 are torn. As a result, an electrolyte solution WT retained in the electrolyte solution retention unit 111 seeps out near the periphery of the electrode 121 and the electrode 122. Similarly, a neutralizer N (for example, citric acid) retained in the neutralizer retention unit 112 seeps out near the periphery of the electrode 121 and the electrode 122.

In step S35, the assistant puts on the disposable diaper 10A to the subject, such as an infant or toddler. The task of putting on the disposable diaper 10A by the assistant is thus complete.

In step S40, the power supply unit 160 (electrification circuit 161) is electrified due to the interposition of the electrolyte solution between the electrode 121 and the electrode 122, and generates electric power.

In step S50, the excretion detection device 100 starts operating as a result of the electric power generated by the power supply unit 160.

In step S60, the radio transmission unit 180 periodically transmits the temperature information acquired by the temperature sensor 130 to the excretion management server 40. Specifically, as described above, the radio transmission unit 180 transmits the temperature information to the excretion management server 40 via the radio relay station 20 and the radio base station 30.

In step S70, based on the received temperature information, the excretion management server 40 determines whether or not the temperature change pattern matches the already-stored pattern. In the present embodiment, the excretion management server 40 stores the temperature change pattern that occurs as a result of urination, and the temperature change pattern that occurs as a result of defecation.

For example, if a temperature change pattern during defecation is compared with that during urination, the rise in the temperature is sharper during urination, and the convergence of temperature change is also faster, as compared to defecation. On the other hand, the rise in the temperature is slow during defecation, and the convergence of temperature change is also slow. Based on such differences in the temperature change pattern, the excretion management server 40 identifies urination and defecation.

When the temperature change pattern based on the received temperature information matches the temperature change pattern of urination or defecation (YES in step S70), then in step S80, the excretion management server 40 transmits an email indicating the occurrence of urination or defecation to a pre-registered address (the cellular phone terminal 50 and the personal computer 60).

Furthermore, in step S85, the neutralizer (citric acid) that has seeped out near the periphery of the electrode 121 and the electrode 122 reduces the pH of the bodily waste (for example, urine), and neutralizes alkalinity of the bodily waste. Specifically, as shown in Fig. 5, when a bodily waste Ex passes through the topsheet 11 and is absorbed by the absorber 13, the neutralizer N that has seeped out near the periphery of the electrode 121 and the electrode 122 comes in contact with the bodily waste Ex. As a result, the alkalinity of the bodily waste Ex is neutralized by the neutralizer N.

In step 90, as a result of receipt of the email, the assistant of the wearer W quickly recognizes urination and defecation by the wearer W, and changes the disposable diaper. In order to prevent interference between the radio signal transmitted by the excretion detection device 100 embedded in the disposable diaper after use, and the radio signal transmitted by the excretion detection device 100 embedded in the new disposable diaper after change, the assistant must take away the disposable diaper after use by more than a fixed distance from the radio relay station 20.

According to the excretion detection device 100 explained above, the neutralizer retention unit 112 is provided at a position where the neutralizer can be in contact with the bodily waste. Specifically, the neutralizer retention unit 112 is provided at the upper side of the electrode unit 120 (electrode 121 and electrode 122). Furthermore, as a result of pressing of the neutralizer retention unit 112, the neutralizer seeps out near the periphery of the electrode unit 120, and the neutralizer comes in contact with the bodily waste. Therefore, the alkalinity of the bodily waste Ex can be neutralized by the neutralizer N.

Thus, the fast corrosion of the electrode unit 120 by urine can be prevented even in a disposable diaper for elderly persons whose pH of urine is often high due to urinary tract infections and the like. Materials such as gold, silver, copper, zinc, aluminum, iron, and carbon, and mixtures of these materials have been disclosed in the prior art documents as materials for electrodes, and the use of two different materials with a large difference in ionization tendencies is highly effective in obtaining the electromotive force. However, in view of using the electrodes in a disposable diaper, a combination of aluminum and silver is desired from the viewpoint of safety and the manufacturing cost, as mentioned in the present embodiment.

However, aluminum is an amphoteric element, and can be dissolved in both acid and alkali. Therefore, as shown below, generally, a lytic reaction with aluminum occurs in a strong base solution.

2 Al + 10 H2O + 2 OH- →- 2 [Al(OH)4(H2O)2]- + 3 H2

Particularly, in elderly persons, for example, when the pH of urine becomes high due to urinary tract infections and the like, aluminum gets dissolved. According to the aforementioned excretion detection device 100, even in such cases, the time until when aluminum gets dissolved can be prolonged.

That is, according to the excretion detection device 100, because relatively low-cost and safe aluminum is used primarily in the electrode 121 and the electrode 122, the manufacturing cost also does not increase substantially. Furthermore, because the time until when the aluminum configuring the electrode 121 and the electrode 122 dissolves is prolonged, a sufficient electrification time can be secured even in the case of contact with urine with a high pH.

In the present embodiment, the electrode unit 120 is provided at a position where the electrode unit 120 can be in contact with the bodily waste of the wearer W and the electrolyte solution retained by the electrolyte solution retention unit 111. Additionally, the electrode unit 120 can also be contact with the electrolyte solution before excretion by the wearer W. Therefore, electrification by the power supply unit 160 is possible even before urination and defecation.

### (5) Other embodiments

So far, the present invention is disclosed through the above embodiments. However, it should not be interpreted that the statements and drawings constituting a part of the present disclosure limit the present invention. From this disclosure, a variety of alternate embodiments, examples, and applicable techniques will become apparent to one skilled in the art.

For example, in the aforementioned embodiment, the neutralizer retention unit 112 is provided at the upper side of the electrolyte solution retention unit 111, however, the positions of the electrolyte solution retention unit 111 and the neutralizer retention unit 112 may be exchanged. Alternatively, the neutralizer retention unit 112 may also be provided adjacent to the electrolyte solution retention unit 111, in a plan view (see Fig. 2 (a)) of the absorber 13 such that the neutralizer retention unit 112 does not overlap the electrolyte solution retention unit 111.

In the aforementioned embodiment, the temperature information is transmitted by using the radio transmission unit 180, however, rather than transmitting the temperature information by the radio transmission unit 180, an alarm such as a buzzer may be operated.

In the aforementioned embodiment, the electrolyte solution retention unit 111 is torn by pressing the electrolyte solution retention unit 111 from the topsheet 11 side, and the electrolyte solution is made to seep out from the electrolyte solution retention unit 111, however, the electrolyte solution may be made to seep out by performing an operation other than pressing. For example, the electrolyte solution retention unit 111 may be torn by pulling thread-like members connected to the electrolyte solution retention unit 111.

Furthermore, the excretion detection device 100 is not just loaded in a diaper, but may also be loaded in an absorbent article such as a sanitary napkin. Additionally, the absorbent article is not just used for human beings, but may also be used for animals, such as pets.

As described above, needless to say, the present invention includes various embodiments and the like not described here. Therefore, the technical range of the present invention is to be defined only by the inventive specific matter according to the adequate claims from the above description.

### [Industrial Applicability]

As described above, according to the present invention, it is possible to provide an excretion detection device by which a sufficient electrification time can be secured even in the case of contact with urine with a high pH, without increasing the manufacturing cost substantially, and also to provide an absorbent article having such an excretion detection device.

### [Explanation of the Reference Signs]

1: excretion management system, 10A, 10B: disposable diaper, 11: topsheet, 12: backsheet, 13: absorber, 20: radio relay station, 30: radio base station, 40: excretion management server, 50: cellular phone terminal, 60: personal computer, 100: excretion detection device, 110: solution and neutralizer retention unit, 111: electrolyte solution retention unit, 112: neutralizer retention unit, 120: electrode unit, 121, 122: electrode, 130: temperature sensor, 150: active tag, 160: power supply unit, 161: electrification circuit, 162: electricity-storage circuit, 170: control unit, 180: radio transmission unit, Ex: bodily waste, N: neutralizer, W: wearer, WT: electrolyte solution

## Claims

1. An absorbent article (10A, 10B) worn by a living body and configured to absorb bodily waste from the living body, wherein
the absorbent article (10A, 10B) comprises:
a topsheet (11) that is in contact with the living body and allows the passage of liquids; and
an absorber (13) configured to absorb the bodily waste,
wherein the absorbent article includes an excretion detection device (100) configured to detect excretion of the bodily waste, and the excretion detection device comprises:
a power supply unit (160) having electrodes (121, 122) configured by using materials with different ionization tendencies;
a neutralizer retention unit (112) configured to retain a neutralizer (N) for reducing the hydrogen ion index of the bodily waste;
a notification unit (180) operated by the electric power generated by the power supply unit, and configured to notify excretion from the living body to outside the excretion detection device; and
a solution retention unit (111) configured to retain an electrolyte solution (WT), wherein
a combination of aluminum and silver is used as the electrodes;
the neutralizer is citric acid, tartaric acid or succinic acid;
the excretion detection device (100) is provided between the topsheet and the absorber; and
the neutralizer retention unit (112) is provided so as to be positioned at the upper side of the electrodes during the use of the excretion detection device.

2. The absorbent article according to claim 1, wherein the neutralizer retention unit (112) is adjacent to the solution retention unit (111).

## Patentansprüche

1. Absorbierender Artikel (10A, 10B), der von einem lebenden Körper getragen wird und zur Absorbierung von Körperausscheidungen aus dem lebenden Körper konfiguriert ist, wobei
der absorbierende Artikel (10A, 10B) Folgendes umfasst:
eine obere Lage (11), die im Kontakt mit dem lebenden Körper ist und den Durchgang von Flüssigkeiten ermöglicht; und
einen Absorber (13), der zur Absorbierung der Körperausscheidungen konfiguriert ist,
wobei der absorbierende Artikel eine Ausscheidungsdetektionsvorrichtung (100) einschließt, die zur Detektion von Ausscheidung der Körperausscheidungen konfiguriert ist, und die Ausscheidungsdetektionsvorrichtung Folgendes umfasst:
eine Stromversorgungseinheit (160), die Elektroden (121, 122) aufweist, die durch Verwendung von Stoffen mit unterschiedlichen Ionisierungstendenzen konfiguriert sind;
eine Neutralisatorhalteeinheit (112), die zum Halten eines Neutralisators (N) für die Reduzierung des Wasserstoffionenindexes der Körperausscheidungen konfiguriert ist;
eine Benachrichtigungseinheit (180), die durch Strom, der von der Stromversorgungseinheit erzeugt wird, betrieben wird und zur Benachrichtigung über eine Ausscheidung von dem lebenden Körper nach außen der Ausscheidungsdetektionsvorrichtung konfiguriert ist; und
eine Lösungshalteeinheit (111), die zum Halten einer Elektrolytlösung (WT) konfiguriert ist, wobei
eine Kombination aus Aluminium und Silber als Elektroden verwendet wird;
der Neutralisator Citronensäure, Weinsäure oder Bernsteinsäure ist;
die Ausscheidungsdetektionsvorrichtung (100) zwischen der oberen Lage und dem Absorber bereitgestellt ist; und
die Neutralisatorhalteeinheit (112) derart bereitgestellt ist, dass sie an der oberen Seite der Elektroden während der Verwendung der Ausscheidungsdetektionsvorrichtung zu positionieren ist.

2. Absorbierender Artikel nach Anspruch 1, wobei die Neutralisatorhalteeinheit (112) benachbart zur Lösungshalteeinheit (111) vorliegt.

## Revendications

1. Article absorbant (10A, 10B) porté par un corps vivant et configuré pour absorber les déchets corporels provenant du corps vivant, dans lequel
l'article absorbant (10A, 10B) comporte :
une feuille supérieure (11) qui est en contact avec le corps vivant et permet le passage de liquides ; et
un absorbeur (13) configuré pour absorber les déchets corporels,
dans lequel l'article absorbant comprend un dispositif de détection d'excrétion (100) configuré pour détecter l'excrétion des déchets corporels, et le dispositif de détection d'excrétion comporte :
une unité d'alimentation en puissance (160) munie d'électrodes (121, 122) configurées en utilisant des matières ayant des tendances à l'ionisation différentes ;
une unité de retenue de neutralisant (112) configurée pour retenir un neutralisant (N) destiné à réduire l'indice ionique de l'hydrogène des déchets corporels ;
une unité de notification (180) actionnée par la puissance électrique générée par l'unité d'alimentation en puissance, et configurée pour notifier l'excrétion provenant du corps vivant à l'extérieur du dispositif de détection d'excrétion ; et
une unité de retenue de solution (111) configurée pour retenir une solution d'électrolytes (WT), dans laquelle
une combinaison d'aluminium et d'argent est utilisée pour faire fonction d'électrodes ;
le neutralisant est de l'acide citrique, de l'acide tartrique ou de l'acide succinique ;
le dispositif de détection d'excrétion (100) est fourni entre la feuille supérieure et l'absorbeur ; et
l'unité de retenue de neutralisant (112) est fournie de façon à être positionnée au niveau du côté supérieur des électrodes durant l'utilisation du dispositif de détection d'excrétion.

2. Article absorbant selon la revendication 1, dans lequel l'unité de retenue de neutralisant (112) est adjacente à l'unité de retenue de solution (111).
